# EUROPÄISCHE PATENTSCHRIFT

(11) **EP 2 617 375 B1**
(45) Veröffentlichungstag und Bekanntmachung des Hinweises auf die Patenterteilung: **11.09.2019**
(21) Anmeldenummer: 13152042.1
(22) Anmeldetag: 21.01.2013
(51) Int. Cl.: A61B 17/50

(54) **Zeckenhebel**
Tick-removal lever
Levier à tiques

(30) Priorität: 20.01.2012 DE 102012200859
(43) Veröffentlichungstag der Anmeldung: 24.07.2013
(73) Patentinhaber: Meckel-Spenglersan Gmbh, 14163 Berlin (DE)
(72) Erfinder: Beller, Klaus-Dieter, 79341 Kenzingen (DE)
(74) Vertreter: Wehlan, Martin

(56) Entgegenhaltungen:
- EP-A2- 1 925 259
- WO-A1-96/38095
- DE-U1-202009 016 207
- US-A- 4 024 874
- US-A1- 2008 082 111

## Beschreibung

Die Erfindung betrifft einen innovativen Hebel als Fremdkörper-Entfernungshilfe aus der Haut, der ein Herausdrehen des Fremdkörpers verhindert und den Fremdkörper definiert aushebelt. Das Instrument eignet sich zum Aushebeln von Gegenständen (Fremdkörpern), insbesondere von Zecken, aus der Haut von Tieren oder Menschen. Es ist in Anlehnung an das Hebelwerkzeug "Geisenfuß", "Kuhfuss", "Nagelzieher" oder "Brechstange" gestaltet. Die Erfindung ist explizit nicht ausgelegt, um Zecken oder andere Fremdkörper "herauszudrehen", sondern um diese unter Vorspannung auszuhebeln. Zusätzlich wird das Vorspannen der Haut genutzt, um Fremdkörper besser und effektiver mit den bekannten medizinischen Hilfsmitteln (Pinzette, Zange, Kanüle) aus der Haut zu entfernen.

### Stand der Technik

Ähnliche Instrumente sind aus dem Stand der Technik bekannt. So wird beispielsweise in der Patentschrift EP 0821571 B1 ein solcher einfacher Geisenfuß beschrieben. Hier wird Wert auf Drehwinkel, Achsstellung und Gabelform gelegt. Bevorzugt wird hier das Entfernen durch Drehen propagiert.

Verschiedene Vorrichtungen zur Zeckenentfernung sind seit langem aus der Praxis bekannt und existieren in den unterschiedlichsten Formen und Größen. Dabei sind die Vorrichtungen häufig in Form von Pinzetten, Haken oder Karten ausgebildet. Es werden auch Zeckenentferner mit integriertem Kältespray beschrieben. Ziel ist immer, eine vollständige Entfernung einer Zecke zu erreichen, ohne das Mundwerkzeug vom Zeckenkörper abzureißen. Auch ist ein quetschen des Zeckenkörpers zu vermeiden, damit kein infektiöses Magensekret der Zecke in den Wirtskörper (Mensch, Tier) injiziert wird.

Aus der EP 1 082 942 B1 ist eine Vorrichtung zum Entfernen vom Zecken bekannt, wobei es sich dabei im Konkreten um eine Zeckenzange mit einer entsprechenden Greifeinrichtung handelt. Die Zange weist eine komplizierte Konstruktion auf und ist recht unhandlich.

Aus den deutschen Gebrauchsmusterschriften DE 297 22 310 U1 und DE 202 21 252 U1 ist ein Zecken-Ziehgerät bekannt, welches aus einem formstabilen steifen Flachmaterial in (Scheck) Kartenformat besteht. Je nach Ausführungsbeispiel sind ein oder zwei Aufnahmeschlitze vorgesehen. Wahlweise sind diverse Zeckenentferner mit integrierter Lupe ausgestattet. Auch das US-Patent A-5,447,511 beschreibt einen Zeckenentferner im Kartenformat. WO96/38095 A1 offenbart eine Zeckenentfernungsvorrichtung, die die Grundlage des Oberbegriffs von Anspruch 1 bildet.

Aufgabe der vorliegenden Erfindung ist es, die aus dem Stand der Technik bekannten eingangs genannten Instrumente grundlegend zu verbessern.

Diese Aufgabe wird erfindungsgemäß - wie in den Patentansprüchen beschrieben - gelöst. Bei der erfindungsgemäßen Vorrichtung handelt es sich um ein Werkzeug mit hoher Funktionalität in Bezug auf die zugrunde liegende Aufgabe. Das erfindungsgemäße Instrument ist vorzugsweise als doppelseitiger "Zeckenzieher" ausgelegt. Die Wirkungsweise der jeweiligen Seite - große oder kleine Zecken / Fremdkörper - ist analog zum Nagelzieher, respektive "Kuhfuss"), wobei die Endstücke in der Form primär gleich sind, aber verschiedene Bauhöhen des Vorspannkufen aufweisen. Es ist aber auch möglich, das erfindungsgemäße Instrument als einseitigen Zeckenentferner zu gestalten. Die Endstücke sind als V-förmiger Spalt (2, 3) ausgebildet, der die Zecke beim Vorschieben des Instruments untergreift und fixiert. Die hautseitigen Flächen der Endstücke mit dem V-förmigen Spalt sind kufenförmig ausgebildet. Als weiteres Hilfsmittel ist eine Lupe in das Instrument integriert.

Das erfindungsgemäße Instrument besitzt gegenüber dem Stand der Technik folgende Vorteile: Die Haut wird vorgespannt und die Fixierung erleichtert
Drehen der Zecke / Fremdkörper wird verhindert
Größenvariabel (für große und kleine Fremdkörper)
Gute Ergonomie, einfaches Handling
Einfacher und kostengünstiger Formenbau
Einfache und kostengünstige Herstellung
Nutzbar als Werbeträger.

Arbeitsweise: Wird das Gerät kräftig hinter der Zecke oder dem Fremdkörper auf die Haut gedrückt, wird die Haut zwischen den Vorspannkufen (4, 5) vorgespannt und aufgeworfen. Somit ist die Zecke / Fremdkörper fixiert und die "Gabel" kann bequem und sicher unter den Zeckenkörper geschoben werden. Durch Kippen des Griffstückes wird die Zecke über die Drehachse ausgehebelt. Über die Vorspannkufen, die über die Drehachse auslaufen, ist ein Herausdrehen und Verdrehen der Zecke gezielt verhindert. Das Vorgehen bei Fremdkörpern (Splitter, Dorne etc.) erfolgt analog. Der so fixierte Bereich kann mit Zange, Pinzette oder Kanüle bearbeitet werden.

In besonderen Fällen kann der Zeckenhebel auch als Mundspatel genutzt werden . Die Zunge wird breitflächig fixiert und zwischen den Vorspannkufen kann z.B. ein Winkelspiegel (HNO) oder eine Kanüle (Neuraltherapie) geführt werden . Auch die Freihaltung der Atemwege (Notfallmedizin) ist mit dem erfindungsgemäßen Instrument möglich .

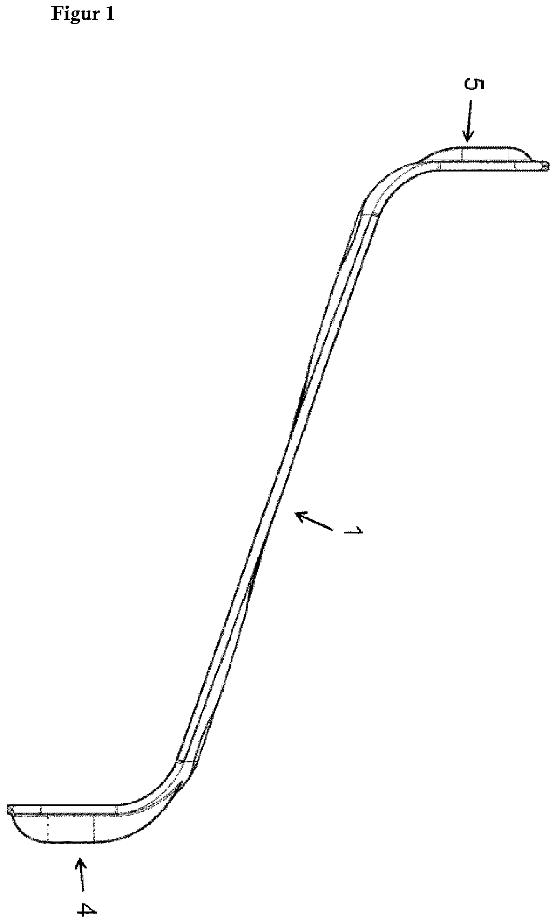

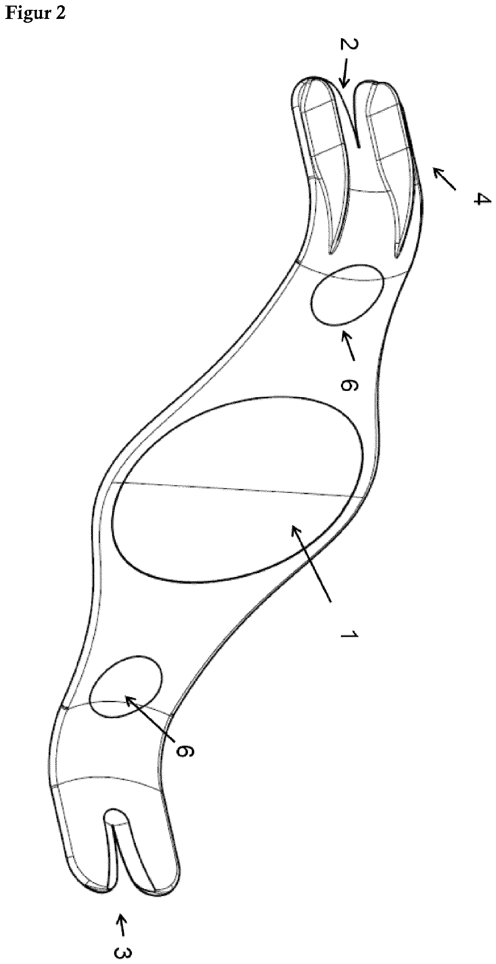

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgenden Figuren eines Ausführungsbeispiels der Erfindung zu verweisen. Nachstehend wird die Erfindung anhand von einem Ausführungsbeispiel (Figur 1 und Figur 2) unter Bezug auf Zeichnungen näher - ohne sich durch die Wahl der abgebildeten Beispiele unnötig beschränken zu wollen - dargestellt.

Ein Ausführungsbeispiel der erfindungsgemäßen Erfindung zeigt Figur 1 und Figur 2.

Figur 1 zeigt eine schematische Seitenansicht einer ersten Ausführungsform des erfindungsgemäßen Instruments. Figur 2 zeigt eine perspektivische Ansicht einer zweiten Ausführungsform des erfindungsgemäßen Instruments.

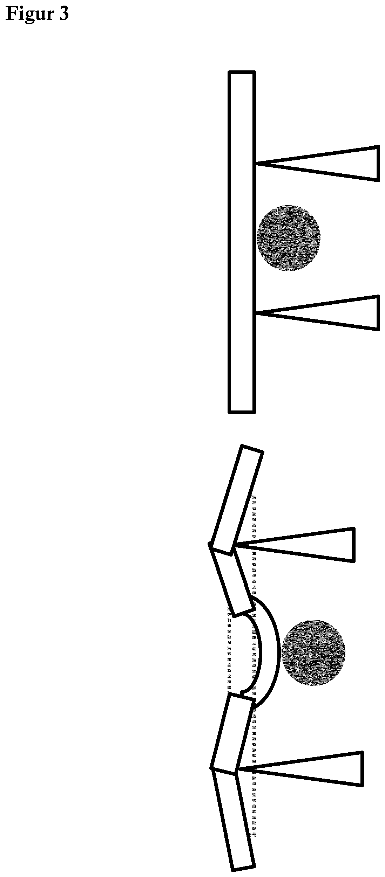

Das gesamte Instrument, einschließlich der Vorspannungskufen, besteht vorzugsweise aus Kunststoff (Spritzguss). Unter dem Begriff "Vorspannungskufen" sind Erhöhungen zu verstehen, die sich an beiden Enden des erfindungsgemäßen Hebels (neben dem V-förmigen Spalt) befinden. Eine bevorzugte Ausführungsform der Erfindung wird erreicht, wenn die Vorspannungskufen parallel verlaufen. Die Kufenbreite kann unterschiedlich sein. Allerdings sollte die Sockelbreite der Kufe nicht das Maß der Kufenhöhe überschreiten. Die Kufen haben eine enorme Bedeutung für ein erfolgreiches Entfernen des Gegenstands aus der Haut. Die Kufen drücken sich in die Haut und stülpen die Haut mit dem Gegenstand oder Fremdkörper, z.B. eine Zecke, in den V-förmigen Fixierspalt (siehe Figur 3).

Deshalb darf der Kufenabstand auch nicht zu klein sein. Zusätzlich verhindert das Eindrücken in die Haut das Drehen des Instruments.

Der Kufenabstand sollte an der kleinen Seite vorzugsweise zwischen 5 und 8 mm und an der großen Seite vorzugsweise zwischen 9 und 12 mm betragen. Gute Ergebnisse werden erreicht, wenn der Kufenabstand an der kleinen Seite 7 mm und an der großen Seite 10 mm beträgt. Diese Werte wurden empirisch über verschiedene Modelle und Versuche entwickelt. Es ist durchaus möglich, dass - in Abhängigkeit vom Hauttyp und Größe des Gegenstands - auch andere Kufenabstände zum Erfolg führen.

Die Kufenhöhen sollten zweckmäßiger Weise an der kleinen Seite 1,2 mm bis 1,9 mm betragen und an der großen Seite 2 bis 4 mm. Bevorzugt sind Kufenhöhen an der kleinen Seite von 1,5 mm und an der großen Seite von 3 mm.

Das erfindungsgemäße Instrument zum Entfernen von Fremdkörpern aus der Haut von Tieren oder Menschen umfasst eine versteifte Griffplatte, die ggf. als Werbefläche nutzbar ist. An einem oder beiden Enden dieser Griffplatte befindet sich eine Krümmung. Diese Krümmung stellt den Drehpunkt dar, über den der Fremdkörper herausgehebelt, aber nicht herausgedreht, werden kann. An diese Krümmung schließt sich der V-förmige Fixierspalt an. An der Seite, die beim Benutzen zur Haut zeigt, sind die Vorspannungskufen enthalten. Diese dienen zum Vorspannen der Haut, um den Fremdkörper durch Hebelung über den Drehpunkt schnell und wirksam zu entfernen.

Gemäß einer bevorzugten Ausführungsform der Erfindung sind die Vorspannungskufen mindestens bis zur Mitte der Krümmung geführt, um das Heraushebeln zu verbessern und ein Herausdrehen des Gegenstands zu verhindern.

Der Begriff "Griffplatte" bedeutet im Zusammenhang mit dieser Erfindung, dass man das erfindungsgemäße Gerät an dieser Stelle zum funktionellen Gebrauch in die Hand nehmen kann. Sie stellt vorzugsweise den flächenmäßig größten Teil der erfindungsgemäßen Vorrichtung dar (siehe Figur 2).

An die Griffplatte schließen sich die Krümmungen an. Die Herstellungsweise einer Griffplatte mit anschließenden Krümmungen sind einem Fachmann bekannt. Unter Begriff "Mitte der Krümmung" ist der Bereich zu verstehen, von dem es zu den ungekrümmten Teilen (Griffplatte und Spaltfläche) gleich weit ist. Die Mitte der Krümmung ist also ein Bereich, der über die gesamte Breite des Gegenstands erstreckt und ist nicht auf einen Mittelpunkt bezogen.

### Bezugszeichenliste

- 1: versteifte Griffplatte, ggf. mit Werbefläche
- 2: V-förmiger Fixierspalt klein
- 3: V-förmiger Fixierspalt groß
- 4: Vorspannungskufen klein
- 5: Vorspannungskufen groß
- 6: Integrierte Vergrößerungslinse

## Patentansprüche

1. Instrument zum Entfernen von Gegenständen oder Fremdkörpern aus der Haut von Menschen oder Tieren umfassend eine Griffplatte (1), an deren einem Ende sich eine Krümmung befindet, an die ein sich ein V-förmiger Fixierspalt (2, 3) anschließt und diese Krümmung einen Drehpunkt darstellt, über den der Gegenstand oder Fremdkörper aus der Haut herausgehebelt werden kann, **dadurch gekennzeichnet, dass** der V-förmige Fixierspalt (2, 3) Vorspannungskufen (4, 5) aufweist, um die Haut vorzuspannen und eine bessere Fixierung des Gegenstands oder Fremdkörpers zu erreichen.

2. Instrument nach Anspruch 1, **dadurch gekennzeichnet, dass** sich an beiden Enden der Griffplatte (1) Krümmungen befinden, an die ein sich verschieden große V-förmige Fixierspalte (2, 3) anschließen.

3. Instrument nach Anspruch 1 oder 2, **dadurch gekennzeichnet, dass** die Vorspannungskufen (4, 5) mindestens bis zur Mitte der Krümmung geführt werden, um das Heraushebeln zu verbessern und ein Herausdrehen des Gegenstands oder Fremdkörpers zu verhindern.

4. Instrument nach einem der Ansprüche 1 bis 3, **dadurch gekennzeichnet, dass** die Vorspannungskufen (4, 5) parallel verlaufen.

5. Instrument nach einem der Ansprüche 1 bis 4, **dadurch gekennzeichnet, dass** der Kufenabstand an der kleinen Seite zwischen 5 und 8 mm, vorzugsweise 7mm und an der großen Seite zwischen 9 und 12 mm, vorzugsweise 10 mm beträgt.

6. Instrument nach einem der Ansprüche 1 bis 5, **dadurch gekennzeichnet, dass** die Kufenhöhe an der kleinen Seite 1,2 mm bis 1,9 mm, vorzugsweise 1,5 mm und an der großen Seite 2 bis 4 mm, vorzugsweise 3 mm beträgt.

7. Instrument nach einem der Ansprüche 1 bis 6, **dadurch gekennzeichnet, dass** Griffplatte ein oder 2 Lupen (Linsen) eingearbeitet sind.

8. Instrument nach einem der Ansprüche 1 bis 7, **dadurch gekennzeichnet, dass** es aus Kunststoff besteht.

9. Instrument nach einem der Ansprüche 1 bis 8, **dadurch gekennzeichnet, dass** es sich bei den zu entfernenden Fremdkörpern um Zecken handelt.

## Claims

1. An instrument for removing an object or a foreign matter from a skin of a human or an animal, comprising a gripping plate (1) at one end of which a curvature is provided to which a V-shaped fixing gap (2, 3) is connected, said curvature representing a pivot point by which the object or foreign matter can be pried out of the skin, **characterised in that** the V-shaped fixing gap (2, 3) comprises bias skids (4, 5) in order to pretension the skin and to reach a better fixation of the object or the foreign matter.

2. The instrument according to claim 1, **characterised in that** curvatures are provided on both ends of the gripping plate (1) to which V-shaped fixing gaps (2, 3) of different size are connected.

3. The instrument according to claim 1 or 2, **characterised in that** the bias skids (4, 5) are guided up to at least the centre of the curvature in order to improve the prying out, and to prevent unscrewing of the object or the foreign matter.

4. The instrument according to any one of claims 1 to 3, **characterised in that** the bias skids (4, 5) run parallel.

5. The instrument according to any one of claims 1 to 4, **characterised in that** the skid distance on the small side is between 5 and 8 mm, preferably 7 mm, and on the large side between 9 and 12 mm, preferable 10 mm.

6. The instrument according to any one of claims 1 to 5, **characterised in that** the skid height on the small side is 1.2 mm to 1.9 mm, preferably 1.5 mm, and on the large side 2 to 4 mm, preferably 3 mm.

7. The instrument according to any one of claims 1 to 6, **characterised in that** one or two magnifying glasses (lenses) are integrated into the gripping plate.

8. The instrument according to any one of claims 1 to 7, **characterised in that** it consists of plastic material.

9. The instrument according to any one of claims 1 to 8, **characterised in that** the foreign matter to be removed are ticks.

## Revendications

1. Instrument pour éliminer des objets ou des corps étrangers de la peau de l'homme ou des animaux comportant une plaque de préhension (1) à une extrémité de laquelle il y a une courbure à laquelle se raccorde une fente de fixation en forme de V (2, 3), et ladite courbure représentant un pivot par lequel l'objet ou le corps étranger peut être extrait de la peau par effet de levier, **caractérisé en ce que** la fente de fixation en forme de V (2, 3) comprend des patins de précontrainte (4, 5) pour précontraindre la peau et pour atteindre une meilleure fixation de l'objet ou du corps étranger.

2. Instrument selon la revendication 1, **caractérisé en ce que** des courbures se présentent aux deux extrémités de la plaque de préhension (1) auxquelles des fentes de fixation en forme de V (2, 3) de différentes tailles se raccordent.

3. Instrument selon la revendication 1 ou 2, **caractérisé en ce que** les patins de précontrainte (4, 5) sont guidés au moins jusqu'au centre de la courbure pour améliorer l'extraction par effet de levier et pour éviter un desserrage de l'objet ou du corps étranger.

4. Instrument selon l'une quelconque des revendications 1 à 3, **caractérisé en ce que** les patins de précontrainte (4, 5) s'étendent parallèlement.

5. Instrument selon l'une quelconque des revendications 1 à 4, **caractérisé en ce que** la distance des patins au petit côté est entre 5 et 8 mm, de préférence 7 mm et au large côté entre 9 et 12 mm, de préférence 10 mm.

6. Instrument selon l'une quelconque des revendications 1 à 5, **caractérisé en ce que** la hauteur des patins est 1,2 mm à 1,9 mm, de préférence 1,5 mm au petit côté, et 2 à 4 mm, de préférence 3 mm au large côté.

7. Instrument selon l'une quelconque des revendications 1 à 6, **caractérisé en ce qu'**une ou deux loupe(s) (lentille(s)) sont intégrées dans la plaque de préhension (1).

8. Instrument selon l'une quelconque des revendications 1 à 7, **caractérisé en ce qu'**il est constitué d'un matière plastique.

9. Instrument selon l'une quelconque des revendications 1 à 8, **caractérisé en ce que** les corps étrangers à éliminer sont des tiques.
